Europäisches Patentamt

European Patent Office

Office européen des brevets

(11) Veröffentlichungsnummer: **0 279 312 B1**

## (12) EUROPÄISCHE PATENTSCHRIFT

(45) Veröffentlichungstag der Patentschrift: **06.05.92**

(21) Anmeldenummer: **88101799.0**

(22) Anmeldetag: **08.02.88**

(51) Int. Cl.⁵: **C07C 201/16**, C07C 205/06

---

(54) **Verfahren zur Abtrennung von Schwefelsäure und Salpetersäure aus bei der Nitrierung von Toluol erhaltenen Dinitrotoluolgemischen.**

---

(30) Priorität: **18.02.87 DE 3705091**

(43) Veröffentlichungstag der Anmeldung:
**24.08.88 Patentblatt 88/34**

(45) Bekanntmachung des Hinweises auf die
Patenterteilung:
**06.05.92 Patentblatt 92/19**

(84) Benannte Vertragsstaaten:
**BE DE ES FR IT**

(56) Entgegenhaltungen:
**US-A- 3 221 064**

(73) Patentinhaber: **BAYER AG**

**W-5090 Leverkusen 1 Bayerwerk(DE)**

(72) Erfinder: **Witt, Harro, Dr.**
**Möhlenbarg 2**
**W-2224 Kuden(DE)**
Erfinder: **Beckhaus, Heiko, Dr.**
**Winterberg 17**
**W-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Abtrennung von Schwefelsäure und Salpetersäure aus bei der Dinitrierung von Toluol mit Mischsäure erhaltenen Schwefelsäure und Salpetersäure enthaltenden Dinitrotoluolgemischen.

Ein allgemein übliches Verfahren zur Herstellung von Dinitrotoluolen besteht darin, daß man zunächst in einer ersten Stufe Toluol mit Salpetersäure in Gegenwart von Schwefelsäure (Mischsäure) nitriert, die erhaltenen Mononitrotoluole aus dem Reaktionsgemisch abtrennt und diese dann in einer zweiten Reaktionsstufe mit Mischsäure weiter zu Dinitrotoluolen nitriert (vgl. z.B. Kirk-Othmer, Encyclopedia of Chemical Technology, 2nd edition, Vol. 13, S. 844 ff). Das nach Abtrennung der Mischsäure erhaltene rohe Dinitrotoluolgemisch enthält noch gelöste oder fein dispergierte Anteile von Salpetersäure und Schwefelsäure. Nach dem Stand der Technik wird dieses rohe Dinitrotoluolgemisch in einer geeigneten Apparatur mit Wasser, gegebenenfalls unter Zusatz von Basen gewaschen (vgl. z.B. US-A-3 221 064), wobei die Säurereste ins Waschwasser und damit ins Abwasser gelangen.

Es wurde nun ein Verfahren zur Abtrennung von Schwefelsäure und Salpetersäure aus bei der Dinitrierung von Toluol mit Mischsäure erhaltenen Schwefelsäure und Salpetersäure enthaltenden Dinitrotoluolen gefunden, das dadurch gekennzeichnet ist, daß man die nach der Abtrennung der Hauptmenge an Schwefelsäure und Salpetersäure erhaltenen Dinitrotoluole, die noch bis zu 6 Gew.-% Schwefelsäure und bis zu 5 Gew.-% Salpetersäure enthalten, mit bis zu 10 Gew.-% Wasser, bezogen auf die Menge an Dinitrotoluolen, vermischt und die sich danach abscheidende Schwefel- und Salpetersäure enthaltende wäßrige Phase abtrennt.

Je nach Art des angewandten Nitrierverfahrens, der Temperatur des rohen Dinitrotoluolgemisches und der Konzentration der in der zweiten Nitrierstufe eingesetzten Mischsäure können in dem rohen Dinitrotoluol-Isomerengemisch, das in etwa 76 bis 77 % 2,4-Dinitrotoluol, 18 bis 19 % 2,6-Dinitrotoluol, 1,3 bis 1,7 % 2,3-Dinitrotoluol, 2,1 bis 2,8 % 3,4-Dinitrotoluol und 0,4 bis 0,8 % 2,5-Dinitrotoluol enthält, Schwefelsäure in einer Menge von bis zu etwa 6 % und Salpetersäure in einer Menge von bis zu etwa 5 % gelöst oder fein dispergiert sein. Dabei ist es möglich, daß die Schwefelsäure und die Salpetersäure sich einander partiell substituierten, d.h., je höher die Konzentration der einen Säure um so niedriger ist die der anderen.

Bevorzugt sind etwa 1 bis 3 % Schwefelsäure und 1 bis 2 % Salpetersäure im rohen Dinitrotoluolgemisch gelöst oder fein dispergiert.

Zur Abtrennung und Wiedergewinnung der im rohen Dinitrotoluolgemisch enthaltenen Schwefel- und Salpetersäure vermischt man das rohe Dinitrotoluolgemisch erfindungsgemäß mit bis zu 10 Gew.-% Wasser, bevorzugt 0,3 bis 8 Gew.-% Wasser, bezogen auf die Menge an Dinitrotoluol.

Durch die geringe Menge an zugemischtem Wasser ist gewährleistet, daß die sich nach dem Vermischen abscheidende Säurephase konzentriert genug ist, um in die erste Nitrierstufe zurückgeführt zu werden. Die zurückgeführte Säurephase kann ohne spezielle Aufkonzentrierung somit in der ersten Nitrierstufe wieder als Mischsäure eingesetzt werden.

Nach dem erfindungsgemäßen Verfahren wird das dem rohen Dinitrotoluolgemisch zugegebene Wasser intensiv mit den Dinitrotoluolen vermischt. Für die Vermischung sind alle gebräuchlichen Mischaggregate geeignet, wie Mischpumpen, die gleichzeitig den Vorteil der Förderung des Reaktionsmediums haben, Rührwerksbehälter, Mischdüsen, Strahldispergatoren, statische Mischer und ähnliche Apparate.

Die Vermischung der Dinitrotoluole mit Wasser wird erfindungsgemäß bei Temperaturen von etwa 65 bis 90°C, bevorzugt bei 65 bis 85°C, durchgeführt.

Neben der einstufigen Abtrennung von Schwefelsäure und Salpetersäure aus dem rohen Dinitrotoluolgemisch ist es nach dem erfindungsgemäßen Verfahren auch möglich, Schwefelsäure und Salpetersäure in zwei oder mehreren Stufen aus dem Dinitrotoluolgemisch abzutrennen.

Dabei kann man so vorgehen, daß man zunächst die Dinitrotoluole mit etwa 0,3 bis 3 Gew.-%, bevorzugt 0,5 bis 1 Gew.-% Wasser, bezogen auf die Menge an Dinitrotoluol, vermischt, die sich abscheidende überwiegend Schwefelsäure enthaltende schwere wäßrige Phase abtrennt und danach die Dinitrotoluole enthaltende organische Phase mit etwa 2 bis 8 Gew.-%, bevorzugt 2 bis 6 Gew.-% Wasser, bezogen auf die Menge an Dinitrotuol, vermischt und die überwiegend Salpetersäure enthaltende leichtere wäßrige Phase abscheidet.

Auch bei dieser zweistufigen Abtrennung von Schwefelsäure und Salpetersäure ist es möglich, die Säurephasen wieder in die erste Stufe der Nitrierung ohne vorherige Aufkonzentrierung zuzugeben.

Die Vorteile des erfindungsgemäßen Verfahrens sind darin zu sehen, daß es möglich ist, die im rohen Dinitrotoluolgemisch vorhandenen Restmengen an Schwefelsäure und Salpetersäure in einer Form zurückzugewinnen, die eine Recyclierung in den Nitrierprozeß möglich macht. Auf diese Weise kann der Verbrauch an Salpetersäure und Schwefelsäure bei der Herstellung von Dinitrotoluol beträchtlich gesenkt werden. Gleichzeitig wird die Nitrat- und Sulfatfracht im Abwasser je nach Verfahrensweise auf bis zu 5 % erniedrigt, wodurch die Umweltbelastung durch Sulfate und Nitrate beträchtlich gesenkt wird.

Die folgenden Beispiele sollen das erfindungsgemäße Verfahren näher erläutern. Alle Prozentangaben in den Beispielen beziehen sich auf Gewichtsprozente.

## Beispiel 1

2.600 kg/h rohes Dinitrotoluol (DNT) wurden kontinuierlich mit 180 kg/h (ca. 7 Gew.-%) vollentsalztem Wasser in einem Behälter von 500 l Inhalt bei 70°C versetzt. Das Gemisch wurde mit einer Kreiselpumpe über einen statischen Mischer umgepumpt und danach einem Koaleszierfilter zugeführt. Der Koaleszierfilter wirkte gleichzeitig als liegende Scheideflasche. Als leichtere wäßrige Phase schieden sich die Säuren (Schwefelsäure und Salpetersäure) ab, die in eine Pumpenvorlage abliefen. Von dort wurden sie zusammen mit aus der zweiten Nitrierstufe zurückgewonnenen Mischsäure in die erste Nitrierstufe zurückgeführt. Das als schwerere organische Phase ablaufende Dinitrotoluol wurde der Dinitrotoluol-Wäsche zugeführt.

Das rohe Dinitrotoluol hatte folgende Isomerenzusammensetzung:

| | |
|---|---|
| 2,4-Dinitrotoluol | 77,46 % |
| 2,6-Dinitrotoluol | 18,64 % |
| 3,4-Dinitrotoluol | 2,19 % |
| 2,3-Dinitrotoluol | 1,16 % |
| 2,5-Dinitrotoluol | 0,55 %. |

Es war verunreinigt durch Spuren von Mononitrotoluol, Trinitrotoluol und Nitrokresolen.

Vor der Vermischung mit Wasser enthielt das rohe Dinitrotoluol 1,07 % Salpetersäure und 5,15 % Schwefelsäure.

Nach Verlassen des Koaleszierfilters waren die Konzentrationen im Dinitrotoluol auf 0,3 % Salpetersäure und 0,21 % Schwefelsäure gefallen. Das bedeutet eine Reduzierung der Abwasserfrachten bei Salpetersäure um ca. 72 % und bei Schwefelsäure um ca. 96 %.

## Beispiel 2

4.000 kg/h rohes Dinitrotoluol mit einer Temperatur von 70°C wurden in einem Behälter von 500 l Inhalt mit ca. 33 kg/h (ca. 0,8 Gew.-%) vollentsalztem Wasser kontinuierlich versetzt. Das Gemisch wurde über einen statischen Mischer umgepumpt und anschließend in einen horizontalen Koaleszierfilter, der gleichzeitig als Scheideflasche wirkte, gefördert. Die schwerere wäßrige Phase, die überwiegend Schwefelsäure enthielt, lief in eine Pumpenvorlage ab. Von dort wurde sie gemeinsam mit der aus der zweiten Nitrierstufe zurückgewonnenen Mischsäure in die erste Nitrierstufe zurückgeführt.

Das als leichtere Phase den Abscheider verlassende Dinitrotoluol wurde erneut, wie oben geschildert, mit 120 kg/h vollentsalztem Wasser (ca. 3 Gew.-%) versetzt und über einen statischen Mischer in einen weiteren Koaleszierfilter geleitet. Die nunmehr leichtere Phase, die überwiegend die Salpetersäure enthielt, lief ebenfalls in die obengenannte Pumpenvorlage ab; von dort gelangte die leichtere wäßrige Phase dann wieder in die erste Nitrierstufe. Das als schwerere organische Phase sich abscheidende Dinitrotoluolgemisch wurde einer Dinitrotoluol-Wäsche zugeführt.

Das eingesetzte rohe Dinitrotoluolgemisch entsprach in seiner Zusammensetzung dem Dinitrotoluolgemisch des Beispiels 1. Es enthielt 1,32 % Salpetersäure und 4,71 % Schwefelsäure. Nach Verlassen der Koaleszierfilter war die Konzentration auf 0,52 % Salpetersäure und 0,15 % Schwefelsäure gefallen. Das bedeutet eine Reduzierung der Nitratfracht des Abwassers um mehr als 60 % und der Sulfatfracht um mehr als 96 %. Es wurden nur 55 % der in Beispiel 1 verwendeten Wassermenge eingesetzt.

## Beispiel 3

In einen Strom von 130 kg/h rohem DNT von 80°C wurde kontinuierlich über eine Düse mit einem Durchmesser von 0,1 mm 1 kg/h vollentsalztes Wasser von ebenfalls 80°C eingedüst. Der Differenzdruck über der Düse betrug 20 bar. Die entstandene Mischung wurde in ein Koaleszierfilter geleitet. Die Verweilzeit der Mischung vor ihrer Trennung betrug ca. 20 Sekunden. Nach der Trennung wurde eine schwerere Säurephase abgezogen. Die leichtere DNT-Phase wurde über eine zweite Düse von 0,2 mm Durchmesser kontinuierlich mit 5 kg/h Wasser versetzt und in einen zweiten Koaleszierfilter geleitet. Der Differenzdruck über der Düse betrug 15 bar. Die Verweilzeit war die gleiche wie in der ersten Stufe. Nach der Trennung lief eine leichtere Säurephase und eine schwerere DNT-Phase ab. Die abgeschiedenen Säurephasen wurden in die erste Stufe der Nitrierung zurückgeführt.

Das rohe DNT enthielt 1,5 % $H_2SO_4$ und 1,3 % $HNO_3$. Das nach der zweiten Stufe ablaufende DNT enthielt noch 0,3 % $H_2SO_4$ (- 77 %) und 0,6 % $HNO_3$ (- 54 %).

Insgesamt wurden bei sehr kurzen Verweilzeiten nur 4,6 % Wasser, bezogen auf das rohe DNT, eingesetzt.

**Patentansprüche**

1. Verfahren zur Abtrennung von Schwefelsäure und Salpetersäure aus bei der Dinitrierung von Toluol mit Mischsäure erhaltenen Schwefelsäure und Salpetersäure enthaltenden Dinitrotoluolen, dadurch gekennzeichnet, daß man die nach der Abtrennung der Hauptmenge an Schwefelsäure und Salpetersäure erhaltenen Dinitrotoluole, die noch bis zu 6 Gew.-% Schwefelsäure und bis zu 5 Gew.-% Salpetersäure enthalten, mit bis zu 10 Gew.-% Wasser, bezogen auf die Menge an Dinitrotoluolen, vermischt und die sich danach abscheidende Schwefel- und Salpetersäure enthaltende wäßrige Phase abtrennt.

2. Verfahren nach Anspruch 1, dadurch gekennzeichnet, daß man die Dinitrotoluole mit 0,3 bis 8 Gew.-% Wasser, bezogen auf die Menge an Dinitrotoluolen, vermischt.

3. Verfahren nach Ansprüchen 1 und 2, dadurch gekennzeichnet, daß man die Vermischung der Dinitrotoluole mit Wasser bei Temperaturen von 65 bis 90°C durchführt.

4. Verfahren nach Ansprüchen 1 bis 3, dadurch gekennzeichnet, daß man zunächst die Dinitrotoluole mit 0,3 bis 3 Gew.-% Wasser, bezogen auf die Menge an Dinitrotoluol, vermischt, die sich abscheidende überwiegend Schwefelsäure enthaltende wäßrige Phase abtrennt und danach die Dinitrotoluole enthaltende organische Phase mit 2 bis 6 Gew.-% Wasser, bezogen auf die Menge an Dinitrotoluol, vermischt und die überwiegend Salpetersäure enthaltende wäßrige Phase abscheidet.

**Claims**

1. A process for separating sulphuric acid and nitric acid from dinitrotoluenes obtained during the dinitration of toluene with mixed acid which contain sulphuric acid and nitric acid, characterised in that the dinitrotoluenes obtained after separation of the majority of sulphuric acid and nitric acid, which still contain up to 6% by weight of sulphuric acid and up to 5% by weight of nitric acid, are mixed with up to 10% by weight of water, based on the quantity of dinitrotoluenes, and the aqueous phase containing sulphuric and nitric acid, which then settles out, is separated.

2. A process according to Claim 1 characterised in that the dinitrotoluenes are mixed with from 0.3 to 8% by weight of water, based on the quantity of dinitrotoluenes.

3. A process according to Claims 1 and 2, characterised in that the dinitrotuenes are mixed with water at temperatures of from 65 to 90°.

4. A process according to Claims 1 to 3, characterised in that the dinitrotoluenes are first mixed with from 0.3 to 3% by weight of water, based on the quantity of dinitrotoluene, the aqueous phase which contains predominantly sulphuric acid and which settles out is separated, and the organic phase containing dinitrotoluenes is then mixed with from 2 to 6% by weight of water, based on the quantity of dinitrotoluene, and the aqueous phase containing predominantly nitric acid settles out.

**Revendications**

1. Procédé pour séparer l'acide sulfurique et l'acide nitrique des dinitrotoluènes obtenus par la dinitration du toluène par le mélange sulfonitrique, contenant de l'acide sulfurique et de l'acide nitrique, caractérisé en ce que les dinitrotoluènes obtenus après la séparation de la majeure partie de l'acide sulfurique et de l'acide nitrique et qui contiennent encore jusqu'à 6% en poids d'acide sulfurique et jusqu'à 5% en poids d'acide nitrique sont mélangés avec 10% en poids d'eau au maximum rapportés à la quantité de dinitrotoluènes, et que la phase aqueuse qui se sépare ensuite et qui contient de l'acide sulfurique et de l'acide nitrique est éliminée.

2. Procédé selon la revendication 1, caractérisé en ce que les dinitrotoluènes sont mélangés avec 0,3 à 8% en poids d'eau rapportés à la quantité de dinitrotoluènes.

3. Procédé selon les revendications 1 et 2, caractérisé en ce que l'on effectue le mélange des dinitrotoluènes avec de l'eau à des températures comprises entre 65 et 90°C.

4. Procédé selon les revendications 1 à 3, caractérisé en ce qu'on mélange tout d'abord les dinitrotoluènes avec 0,3 à 3% en poids d'eau rapportés à la quantité de dinitrotoluènes, la phase aqueuse qui se sépare et qui contient principalement de l'acide sulfurique étant alors séparée et la phase organique contenant les dinitrotoluènes étant ensuite mélangée avec 2 à 6% en poids d'eau rapportés à la quantité de dinitrotoluènes, avec séparation de la phase aqueuse contenant principalement de l'acide nitrique.